Europäisches Patentamt

European Patent Office

Office européen des brevets

(11) Publication number: 0 467 516 A1

# EUROPEAN PATENT APPLICATION

(21) Application number: 91304673.6

(22) Date of filing: 23.05.91

(51) Int. Cl.5: **A61M 25/04**, **A61M 29/04**, **A61B 17/12**

(30) Priority: 20.07.90 US 556042

(43) Date of publication of application:
22.01.92 Bulletin 92/04

(84) Designated Contracting States:
AT BE CH DE DK ES FR GB GR IT LI LU NL SE

(71) Applicant: **Bristol-Myers Squibb Company**
**345 Park Avenue**
**New York, N.Y. 10154(US)**

(72) Inventor: **Weeks, Vaughan Barry**
**7346 W. River Road**
**Caledonia, Wisconsin 53108(US)**
Inventor: **Tessmann, Terri Lynn**
**1329 Spring Valley Drive**
**Racine, Wisconsin 53405(US)**

(74) Representative: **Brown, David Alan et al**
**MATHYS & SOUIRE 10 Fleet Street**
**London EC4Y 1AY(GB)**

(54) Hemostatic stent.

(57) A stent (10) for both maintaining fluid flow through a body passage and controlling bleeding emanating from the walls of the body passage comprises an elongated tube (11) having a lumen (15) so that fluid can pass therethrough and a hygroscopic/hydrophilic material (17) which expands when wetted by water or aqueous body fluids to exert a hemostatic pressure to control bleeding emanating from the walls of the body passage. In one embodiment, the hygroscopic/hydrophilic material (16) is coated on the outer wall of the tube (11). In another, it is in a chamber (20) and in still another the hygroscopic/hydrophilic material (23) is in a portion (22) of the tube (11).

FIG. 3

The present application relates generally to stents or catheters. More particularly, it relates to stents or catheters which help control bleeding from the walls of a body passage.

The use of stents or catheters to maintain fluid flow in body passages is well known. In addition, it is known to use hydrophilic materials to lubricate stents and catheters to make them easier to insert into body passages. In addition, it also is known to control bleeding in body passages, such as nasal passages, by the use of packing which exerts a hemostatic pressure upon the walls of body passages. However, the use of packing usually cuts off normal flow through the body passage thereby creating another type of problem.

It would obviously be desirable to have a stent or catheter that could both provide and maintain flow through a body passage and which would control or stop any bleeding that might otherwise emanate from the walls of the body passage.

It is an object of the present invention to disclose a stent which both maintains flow through a body passage and which controls any bleeding that might emanate from the wall of the body passage.

The stent of the present invention comprises an elongated tubular member having an inlet at one end, an outlet at the other end and a lumen connecting the inlet to the outlet so that fluid can flow through the stent; and, hemostatic means which swells when wetted to exert a hemostatic pressure upon the wall of a body passage to control any bleeding emanating therefrom.

In one embodiment, the hemostatic means includes a hygroscopic/hydrophilic material which is in a coating on the outer wall of the tubular member. In another embodiment, the hygroscopic/hydrophilic material is in a coating on the outer wall which can be wetted by aqueous liquid from the lumen of the tubular member.

In still another embodiment, the hemostatic means includes a tubular elastic sleeve which is positioned concentrically around the outside of the tubular member and is sealed at its ends to the member to form a chamber which contains a hygroscopic/hydrophilic material. The hygroscopic/hydrophilic material can be wetted by aqueous liquid which passes through the elastic sleeve or which leaves the lumen of the tubular member by passing through the wall of the tubular member.

In a further embodiment, the hemostatic means comprises a portion of the tubular member which is comprised of or contains a hygroscopic or hydrophilic material which swells outwardly when wetted. If necessary, the portion of the stent is coated with a hemostatic material or covered with a stretchable hemostatic material.

The stent of the present invention can be made in a wide variety of shapes and sizes and used to maintain fluid flow through a wide variety of body passages including arteries, veins, nasal passages and urinary passages, such as the ureter and urethra. In an especially preferred embodiment, the stent is sized and shaped so that it can be used to maintain fluid flow through the urethra and to control bleeding following a transurethral resection.

The invention will now be described, by way of example, with reference to the accompanying drawings, in which:

Figure 1 is a perspective view of one embodiment of a stent of the present invention;

Figure 2 is taken along lines 2-2 in Figure 1;

Figure 3 is a schematic view showing the stent of Figure 1 in a body passage and exerting a hemostatic pressure upon the walls of the body passage;

Figure 4 is a sectional view taken along the lines 4-4 in Figure 3;

Figure 5 is a partial view, partly in section, of another embodiment of the stent;

Figure 6 is a view like Figure 1 of still another embodiment of the stent;

Figure 7 is a view like Figure 1 of a further embodiment of the stent;

Figure 8 is a view taken along lines 8-8 in Figure 7;

Figure 9 is a view like Figure 3 of the embodiment of Figure 7; and

Figure 10 is a view like Figure 4 of the stent of Figure 7.

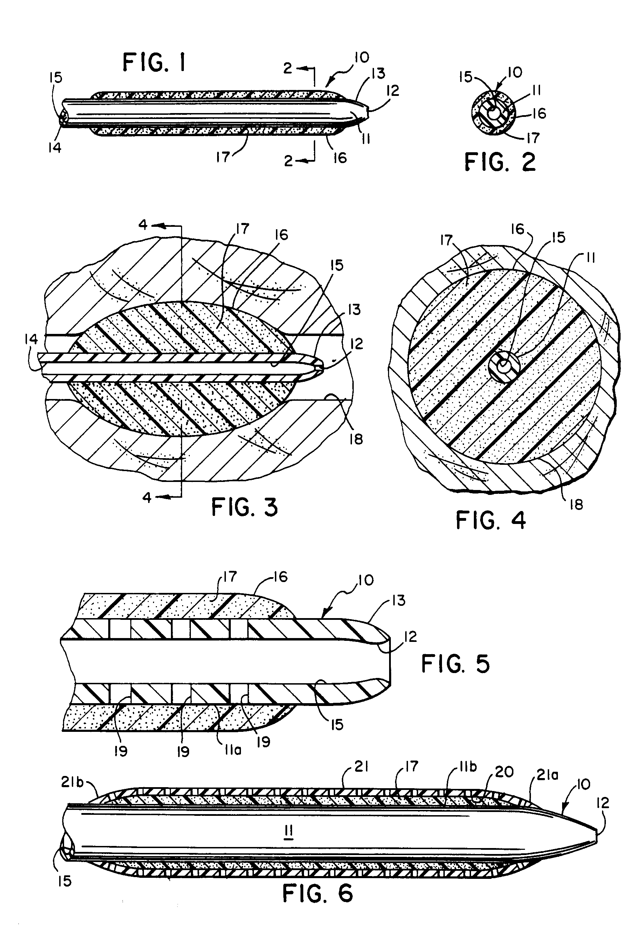

As seen in Figures 1 to 4, the stent 10 of the present invention comprises an elongated tube 11 of a biocompatible material, such as medical grade silicone rubber. The tube 11 has an inlet 12 at one end with a tip 13, an outlet 14 at the other end and a lumen 15 which extends from the inlet 12 to the outlet 14. The tip 13 is rounded to ease the introduction of the stent 10 into a body passage. As seen best in Figures 1 to 4, a portion of the tube 11 has an outer concentric layer or coating 16 of a hygroscopic/hydrophilic material 17.

In Figures 1 and 2, the stent 10 is seen prior to introduction into a body passage. As seen therein, the material 17 is not swollen.

In Figures 3 and 4, the stent 10 is seen in the state it assumes after it has been in the body passage long enough for the material 17 to absorb water and swell and expand into contact with the wall of the body passage 18.

In the embodiment seen in Figure 5, a portion of the wall 11a of the tube 11 beneath the outer layer 16 has passages 19 extending therethrough so that water passing through the lumen 15 can reach the material 17 in the layer 16 and cause it to swell.

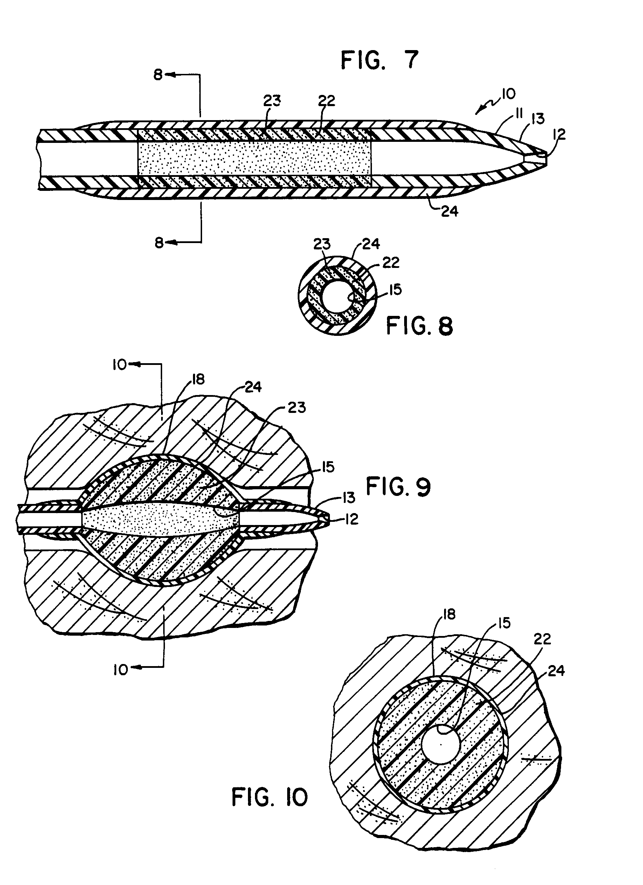

In Figure 6, the material 17 is contained in a chamber 20 which is formed by the outer wall 11b of the tube 11 and a concentric sleeve 21 which surrounds and is sealed at its ends 21a and 21b to the outer wall 11b and the tube 11. The sleeve 21 may be made of an elastic material which is water permeable (eg perforated) so that water can reach the material 17 in the chamber 20. Alternatively, the sleeve 21 can be made of a non water permeable material and water introduced into the chamber 20 through passages like those shown in Figure 5.

In the stent of Figures 7 to 10, the hemostatic means is a portion 22 of the tube 11 which is comprised of or contains a hygroscopic or hydrophilic material 23 and which swells outwardly when wetted by water or a body fluid. In the preferred embodiment seen therein, the portion 22 is covered by a sleeve 24 of a stretchable material having a hemostatic surface, ie., a surface that stops bleeding. In use the stent of Figure 7 functions in a manner similar to that of the stent of Figures 1 to 4.

The embodiments of the invention shown in Figures 1 to 5 can be used with a solid hydrophilic or hygroscopic material of proven biocompatibility where there is no danger of it migrating from the desired location. However, if it is desired to use a hydrophilic material which is a powder or a liquid or a material which is not of proven biocompatibility, the embodiments of Figures 6 to 10 may be preferred.

The stent of the present invention obviously may take other forms than those described; however, in any case the tubular member 11 of the stent 10 should be capable of maintaining a usable lumen for the passage of fluid even when the hydrophilic material has expanded. A suitable material for both the tubular member 11 and the elastic sleeve 21 might be silicone rubber.

The preferred hygroscopic/hydrophilic material is a solid, water-swellable, organic polymeric hydrocolloid which is insoluble in water but which is capable of absorbing water, including water from a body fluid. Suitable materials are swellable gels of the type disclosed in US patent no. 4 138 382 or the materials of US patent nos. 4 424 305 and 4 914 066, all of which are incorporated by reference herein. The hygroscopic/hydrophilic material may be combined with other materials, such as binders, to form the outer coating 16 seen in Figures 1 to 5; it may take the form of a powder or a liquid which would be contained in the chamber 20 of Figure 6; and, it may comprise or be contained in the tubular member 22 of Figures 7 to 10.

In the preferred method of use, the rounded tip 13 of the stent 10 is introduced into a body passage and the stent 10 simply advanced to the desired location. The hygroscopic/hydrophilic material is then wetted, either passively by body fluids in the stent of Figures 1 to 4 or passively and/or actively with body fluids and/or an aqueous liquid from the lumen 15 in the stents of Figures 5, 6 and 7 to 10. When the stent 10 is no longer needed, it can be removed from the body passage. When the stent is of a bioabsorbable material, it can be left in place until it has been absorbed, if desired.

It will be readily apparent to those skilled in the art that a variety of changes and modifications can be made without departing from the scope of the invention.

## Claims

1. A stent for maintaining fluid flow through a body passage and controlling bleeding emanating from the walls of said passage, said stent comprising:

   a. an elongated tubular member having an inner wall and an outer wall, said member having an inlet adjacent one end and an outlet at the other end, and a lumen so that fluid can flow from the inlet to the outlet; and,

   b. hemostatic means which swells when wetted to exert a hemostatic pressure on the wall of said body passage to control any bleeding emanating therefrom.

2. A stent as claimed in Claim 1 in which the hemostatic means is a layer of hygroscopic/hydrophilic material on the outer wall of the tubular member.

3. A stent as claimed in Claim 1 in which the hemostatic means is a layer of hygroscopic/hydrophilic material which covers a moisture permeable portion of the outer wall of the tubular member.

4. A stent as claimed in Claim 1 in which the hemostatic means is a hygroscopic/hydrophilic material contained in a chamber formed by an elastic sheath with a water permeable wall and the outer wall of the tubular member.

5. A stent as claimed in Claim 1 in which the hemostatic means is a portion of the tubular member.

6. A stent for maintaining fluid flow through a body passage and controlling any bleeding emanating from the walls of said passage, said stent comprising:

   a. an elongated tubular member having an inlet at one end and an outlet at the other end, and a lumen extending from the inlet to the outlet so that fluid can flow therethrough;

b. an elastic cover concentric with such tubular member and sealed thereto to form a chamber; said chamber being permeable to water; and

c. a hygroscopic/hydrophilic material in said chamber, which expands when wetted.

FIG. 1

FIG. 2

FIG. 3

FIG. 4

FIG. 5

FIG. 6

EP 0 467 516 A1

FIG. 7

FIG. 8

FIG. 9

FIG. 10

European
Patent Office

**EUROPEAN SEARCH
REPORT**

Application Number

**EP 91 30 4673**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 911 308  (BRENNAN) <br> * abstract; figures * * page 8, line 21 - line 28 * * page 9, line 25 - page 10, line 4 * <br> – – – | 1-3,5 | A 61 M 25/04 <br> A 61 M 29/04 <br> A 61 B 17/12 |
| Y | GB-A-2 185 400  (TAYLOR) <br> * abstract; figures * * page 3, line 107 - page 4, line 1 * <br> – – – | 1-3,5 | |
| A | EP-A-0 227 230  (MENLO CARE, INC.) <br> * abstract; claim 13; figure 2 * <br> – – – | 1-6 | |
| A | DE-A-2 709 302  (ZEPPELIN) <br> * claim 2; figures * <br> – – – | 1,6 | |
| A | EP-A-0 193 406  (MEDTRONIC, INC.) <br> * abstract; figure 2; table 1 * <br> – – – – – | 1,2,6 | |

|  |  |  | TECHNICAL FIELDS SEARCHED (Int. Cl.5) |
|---|---|---|---|
|  |  |  | A 61 M <br> A 61 F <br> A 61 B |

The present search report has been drawn up for all claims

| Place of search | Date of completion of search | Examiner |
|---|---|---|
| The Hague | 10 October 91 | ZEINSTRA H.S.J.H. |